# EUROPEAN PATENT APPLICATION

(11) **EP 1 915 990 A1**
(43) Date of publication of application: **30.04.2008**
(21) Application number: 06781117.4
(22) Date of filing: 14.07.2006
(51) Int. Cl.: A61K 9/24, A61K 47/10, A61K 47/12, A61K 47/14, A61K 47/26, A61K 47/32, A61K 47/34, A61K 47/36, A61K 47/38, A61K 47/42, A61K 47/44, G01N 33/15, A61K 31/341, A61K 31/496

(54) **SUSTAINED RELEASE PREPARATION**

(30) Priority: 15.07.2005 JP 2005206815; 15.07.2005 JP 2005206826; 14.12.2005 JP 2005360038; 14.12.2005 JP 2005360040
(71) Applicant: Kissei Pharmaceutical Co., Ltd., Matsumoto-shi, Nagano 399-8710 (JP)
(72) Inventor: ISSHIKI, N. Central Res. Lab., Kissei Pharmac. Co., Azumino-shi, Nagano;3998304 (JP); KAMADA, N. Central Res. Lab.,Kissei Pharmac. Co., Azumino-shi, Nagano 3998304 (JP); TAKEUCHI, H. Central Res. Lab., Kissei Pharmac. Co, Azumino-shi, Nagano; 3998304 (JP)
(74) Representative: Hayes, Adrian Chetwynd
(86) International application number: PCT/JP2006/314043
(87) International publication number: WO 2007/010847

(57) **Abstract**

The invention provides a preparation which shows a satisfactory gastric residence time, has such a size that allows for easy ingestion, can quickly disintegrate after expelled from the stomach, and can be prepared readily in an industrial scale. A gastric retentive preparation having a gastric resident layer and a drug release layer is provided, wherein the gastric resident layer does not disintegrate in the stomach and disintegrates in the intestine. Preferably, the gastric resident layer has a minimum diameter of 7 mm or more as measured after stirring the preparation in the first fluid at 200 rpm at 37° C for 15 hours under the conditions of the paddle method in the dissolution test in accordance with Japanese Pharmacopoeia and has a maximum diameter of 6 mm or less as measured after further stirring the preparation in the second fluid at 200 rpm at 37°C for 9 hours under the same conditions.

## Description

### TECHNICAL FIELD

The present invention relates to a gastric retentive preparation, a method for determining gastric residence properties, and a method for controlling a gastric residence time. More specifically, the invention relates to a gastric retentive preparation including a gastric resident layer that has a sufficient residence time in the stomach and that quickly disintegrates in the intestine, a method for determining gastric residence properties through measurement of mechanical strength of the gastric retentive preparation, and a method for controlling a gastric residence time.

### BACKGROUND ART

There has been a development of gastric retentive preparations as preparations that are intended to control release of drugs in the stomach. Further studies on such preparations are underway. Various types of gastric retentive preparations are known, including a mucosa-adhesion type, a unique-shape type, a swelling type, and a floating type, for example.

The gastric retentive preparation of a mucosa-adhesion type is intended to prolong the gastric residence time by inclusion therein of a mucosa-adherent substance that allows the preparation to adhere to the mucosa of the stomach. However, the preparation cannot provide a sufficient gastric residence time because it does not readily adhere to the wall of a rapidly moving stomach and readily detaches itself from the mucosa by the metabolic turnover of the mucosa. There is also a problem of safety, such as a concern over the irritant effect of the preparation on the stomach mucosa.

The gastric retentive preparation of a unique-shape type is designed to elongate and extend at a particular temperature, pH, or other conditions of the stomach environment so that it takes the unique shape of, for example, crossed rods or a windmill that permits gastric retention. However, owning to the unique shape, the preparation is difficult to prepare and has the risk of damaging the stomach mucosa or preventing passage of food.

The gastric retentive preparation of a swelling type is intended to extend the gastric residence time with use of an ingredient that swells upon contact with water and thereby increases the preparation to a size that makes passage through the pylorus difficult. In the gastric retentive preparation of a swelling type, the attempt to prevent passage through the pylorus is made by increasing the size of preparation, which is achieved by the swelling of the preparation. Because of this, the preparation intrinsically lacks the mechanical strength necessary to resist the mechanical movement, such as contraction, of the stomach. Therefore, the preparation, when swollen, readily undergoes erosion and reduces its size. Further, because release of a drug is controlled by the erosion of the preparation, control of drug release is difficult when the preparation is designed to erode slowly, whereas, when designed to erode quickly, the preparation reduces its size and is easily expelled from the stomach. For this reason, the gastric retentive preparation of a swelling type is greater in size than other types of gastric retentive preparations, which makes the preparation difficult to ingest.

Recently, a gastric retentive preparation of a swelling type has been reported in which the bilayer structure formed of a drug layer and a swelling layer allows the drug releasing property and the swelling property to be independently controlled (see Patent Literature 1). However, assessment of the preparation by the inventors of the present invention revealed that the preparation still had the conventional problem; namely, the preparation has poor mechanical strength after swelling and readily undergoes erosion, and cannot provide a sufficient gastric residence time when it has an easily ingestible size, whereas ingestion is difficult when the preparation is designed to provide a sufficient gastric residence time (see Comparative Example 1).

There has also been a report of a preparation in which a portion of a polymer matrix that swells upon contact with the gastric fluid is surrounded by an insoluble band that prevents the covered portion of the polymer matrix from swelling and thereby provides sufficient rigidity (mechanical strength) to withstand stomach contractions and delay expulsion of the preparation from the stomach (see Patent Literature 2). However, complicated procedures are required to surround a portion of the polymer matrix with the insoluble band, and preparation of such preparations on industrial scale is difficult to achieve.

The gastric retentive preparation of a floating type is intended to extend the gastric residence time by causing the preparation to float in the stomach. However, because it takes time before the orally administered preparation floats, there are cases where the preparation is expelled from the stomach before it can float in the stomach.

To solve this problem, a gastric retentive preparation of a floating type has been proposed that has a bilayer structure including a layer formed of a drug additive of a low bulk density (floating layer) and a drug-release control layer (see Patent Publication 3). The floating layer of the preparation includes a low-density high-bulk cellulose derivative such as ethyl cellulose and provides a sufficient gastric residence time (see Comparative Example 2). However, since most of the floating layer is not eroded in the body, there is a problem that the digestive tract may be damaged, among others. Further, since the floating layer is excreted with the feces in its original form, it may give patients an uneasy feeling.
Patent literature 1: JP-A-2005-132803
Patent literature 2: JP-T-2001-527023 (the term "JP-T" as used herein means a published Japanese translation of a PCT patent application)
Patent literature 3: International Publication No. WO 2004/002445 pamphlet

### DISCLOSURE OF THE INVENTION

An object of the present invention is to provide a preparation which has a sufficient gastric residence time, has a size that enables easy ingestion, can disintegrate quickly after expelled from the stomach, and can be readily prepared in an industrial scale. The invention also provides a method for determining gastric residence properties, and a method for controlling a gastric residence time.

The inventors of the present invention diligently worked to achieve the foregoing object and found that a gastric residence time could be controlled with use of a gastric resident layer, unclassified into any known categories of gastric resident layers, that has sufficient retentive properties owning to the retention of its shape for an extended time period in the stomach, and that has desirable disintegrating properties in the intestine. The present inventors further found that the gastric residence time could be controlled by controlling mechanical strength of preparations, which is achieved by varying the type and amount of ingredients included in the gastric resident layer. The present invention was accomplished based on these finding.

Specifically, a gist of the present invention lies in a gastric retentive preparation including a gastric resident layer and a drug releasing layer, wherein the gastric resident layer does not disintegrate in the stomach and disintegrates in the intestine, a method for determining gastric retentive properties through measurement of mechanical strength of the gastric retentive preparation, and a method for controlling a gastric residence time.

### BRIEF DESCRIPTION OF THE DRAWINGS

[Fig. 1] Fig. 1 is a diagram illustrating an apparatus used for strength test.

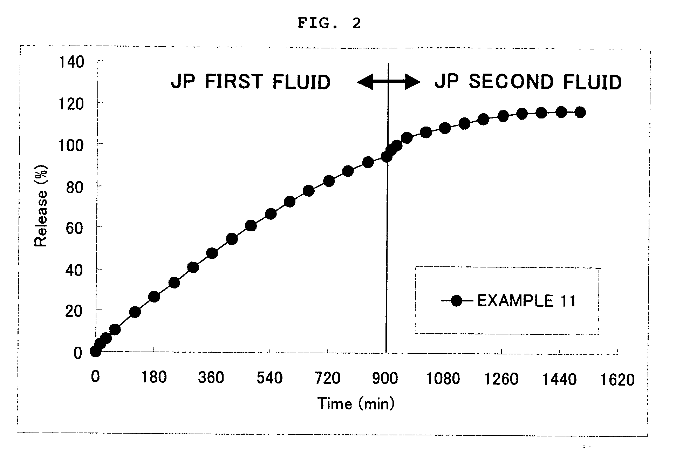

[Fig. 2] Fig. 2 is a diagram representing solubility of ciprofloxacin contained in a gastric retentive preparation of Example 11, in which the vertical axis represents percent release of ciprofloxacin, and the horizontal axis represents a time from the start of dissolution test.

### BEST MODE FOR CARRYING OUT THE INVENTION

A gastric retentive preparation of the present invention includes a gastric resident layer and a drug releasing layer, wherein the gastric resident layer does not disintegrate in the stomach and disintegrates in the intestine.

As used herein, the term "does not disintegrate in the stomach" means that the gastric resident layer is not eroded by the gastric fluid or the contraction motion of the stomach and the shape thereof remains stable. The gastric retentive preparation having the gastric resident layer that does not disintegrate in the stomach is, for example, one in which the gastric resident layer has a minimum diameter (hereinafter referred to as "small diameter") of 7 mm or more, and more preferably 8 mm or more, which makes passage through the pylorus difficult, as measured after stirring the preparation in the first fluid at 200 rpm at 37°C for 15 hours under the conditions of the paddle method in the dissolution test in accordance with the Japanese Pharmacopoeia, Fourteenth Edition (hereinafter referred to as "JP").

Preferably, the preparation has mechanical strength against the mechanical action such as the contraction motion of the stomach, because the preparation is subjected to such action in the stomach in addition to the erosion by the gastric fluid. An example of a preparation with mechanical strength is one that can retain its shape in a test simulating the mechanical action of the stomach, or more specifically one that has a maximum load of 5000 g or more when compressed by 60 mm per minute to a thickness of 1 mm after being rotated at 200 rpm at 37°C for 10 hours in a horizontally laid 50 mL volume conical tube (30×115 mm) containing 50 g of glass beads having an outer diameter of about 4 mm to 5 mm and 30 mL of the JP first fluid, the JP first fluid being replaced every 2.5 hours during the rotation. A preparation with a maximum load below 5000 g readily undergoes erosion and may reduce to a size small enough to pass through the pylorus. It is therefore preferable that the preparation have a maximum load of 10000 g or more. The preparation after the test preferably has a small diameter of 7 mm or more, and more preferably 8 mm or more, which makes passage through the pylorus difficult.

The term "disintegrates in the intestine" means that the gastric resident layer quickly disintegrates in the intestinal fluid. An example of a gastric resident layer that disintegrates in the intestine is one that has a maximum diameter (hereinafter referred to as "large diameter") of 6 mm or less as measured after the preparation, taken out of the first fluid in which the preparation had been stirred under the conditions of the paddle method in the dissolution test in accordance with JP, was stirred in the second fluid at 200 rpm at 37°C for 9 hours under the same conditions. It can be said that the preparation has better disintegrating properties when the gastric resident layer has smaller large diameters in the test. The preparation have preferably a large diameter of 4 mm or less, and more preferably 2 mm or less. A preparation with the gastric resident layer that completely disintegrates is most preferable.

An example of ingredients used for preparing the gastric resident layer that does not disintegrate in the stomach and disintegrates in the intestine is a polymer ingredient that shows higher solubility or swellability in weakly acidic to weakly alkaline medium such as the intestinal fluid (pH 5 to 8) than in acidic medium such as the gastric fluid (pH 1 to 3) (hereinafter such polymer ingredient will be referred to as "pH-dependent polymer ingredient"). Examples of pH-dependent polymer ingredients include an enteric ingredient; and a polymer ingredient selected from the group consisting of carboxymethyl celluloses, polyacrylic acids, and polysaccharides having a carboxyl group or a sulfo group (hereinafter such polymer ingredient will be referred to as "acidic functional group-containing polymer ingredient").

Any enteric ingredient may be used as long as it is a substance commonly used for enteric preparations and that dissolves or swells in an aqueous solution at a pH of 5.5 or greater. Examples of enteric ingredients include enteric cellulose derivatives such as cellulose acetate phthalate, cellulose acetate succinate, methyl cellulose phthalate, hydroxymethylethyl cellulose phthalate, hydroxypropylmethyl cellulose phthalate, hydroxypropylmethyl cellulose acetate succinate, cellulose acetate trimellitate, and carboxymethylethyl cellulose; enteric acrylic acid copolymers such as a methyl methacrylate-methacrylic acid copolymer, a styrene-acrylic acid copolymer, a methylacrylate-methacrylic acid-octyl acrylate copolymer, a methacrylic acid-ethylacrylate copolymer (commercially available as Eudragit L100-55, and Eudragit L30D-55), a methylacrylate-methylmethacrylate-methacrylic acid copolymer (commercially available as Eudragit FS30D), and a methacrylic acid-methylmethacrylate copolymer (commercially available as Eudragit L100, and Eudragit S100); enteric polyvinyl derivatives such as polyvinyl butyrate phthalate and polyvinyl acetoacetal phthalate; and enteric maleic acid-vinyl copolymers, such as a copolymer of vinyl acetate and maleic acid anhydride, a copolymer of vinylbutyl ether and maleic acid anhydride, and a copolymer of styrene and maleic acid monoester. Among these, the enteric cellulose derivatives and enteric acrylic acid copolymers are preferable. The enteric ingredient may be used either individually or in a combination of two or more kinds with any proportions.

Examples of carboxymethyl celluloses include carmellose, carmellose calcium, carmellose sodium, and croscarmellose sodium. Examples of polyacrylic acids include carboxyvinylpolymer and polycarbophil calcium. Examples of polysaccharides having a carboxyl group or a sulfo group include sodium carboxymethyl starch, alginic acid, xanthan gum, gellan gum, hyaluronic acid, carrageenan, chondroitin sulfate, and dextran sulfate. Among these, the carboxymethyl celluloses and polyacrylic acids are preferable. The acidic functional group-containing polymer ingredient may be used either individually or in a combination of two or more kinds with any proportions, or in further combination with the enteric ingredient.

When solubility or swellability of the pH-dependent polymer ingredient is high, there are cases where the gastric retentive preparation readily disintegrates. Such tendency is prominent when the acidic functional group-containing polymer ingredient is used as an ingredient. In this case, the gastric retentive preparation that retains its shape in the stomach and shows a disintegrating property in the intestine, can be realized by inclusion of a hydrophobic ingredient that exhibits a moderate strength in water.
Examples of such hydrophobic ingredients include: cellulose derivatives such as ethyl cellulose and cellulose acetate; acrylic acid copolymers such as an ethylacrylate-methylmethacrylate copolymer (commercially available as Eudragit NE), and an ethyl acrylate-methyl methacrylate-trimethylammonioethyl methacrylate chloride copolymer (commercially available as Eudragit RL, and Eudragit RS); vinyl acetate polymers such as an ethylene-vinyl acetate copolymer, and vinyl acetate resin; oils and fats, such as hydrogenated oil, whale wax, bee wax, carnauba wax, lanolin, and cacao butter; higher fatty acids such as capric acid, lauric acid, myristic acid, palmitic acid, stearic acid, arachic acid, and behenic acid; higher fatty acid esters, such as higher fatty acid alkyl esters, (poly)glycerol higher fatty acid esters, higher fatty acid sorbitans, higher fatty acid polyethylene glycols, and sucrose higher fatty acid esters; higher alcohols such as lauryl alcohol, myristyl alcohol, cetyl alcohol, stearyl alcohol, and behenyl alcohol; hydrocarbons such as polyethylene, polystyrene, polyisobutylene, and microcrystalline wax; polycaprolactone; polylactate; polyglycolate; and polyamides.
Examples of higher fatty acid alkyl esters include: myristyl myristate, cetyl palmitate, cholesteryl stearate, and batyl monostearate.
Examples of (poly)glycerol higher fatty acid esters include: glyceryl myristate, glyceryl monostearate, glyceryl distearate, diglyceryl monostearate, tetraglyceryl monostearate, tetraglyceryl tristearate, tetraglyceryl pentastearate, hexaglyceryl monostearate, hexaglyceryl tristearate, hexaglyceryl tetrabehenate, decaglyceryl monostearate, decaglyceryl distearate, decaglyceryl tristearate, decaglyceryl pentastearate, decaglyceryl pentahydroxystearate, decaglyceryl heptastearate, and decaglyceryl decastearate.
Examples of higher fatty acid sorbitans include: sorbitan monopalmitate, sorbitan monostearate, sorbitan sesquistearate, and sorbitan tristearate.
Examples of higher fatty acid polyethylene glycols include: ethylene glycol monostearate, polyethylene glycol monostearate, ethylene glycol distearate, diethylene glycol stearate, and polyethylene glycol distearate.
Examples of sucrose higher fatty acid esters include: sucrose stearate ester, sucrose palmitate ester, sucrose oleate ester, sucrose laurate ester, sucrose behenate ester, and sucrose erucate ester.
Among these, the cellulose derivatives or oils and fats are preferable. The hydrophobic ingredient may be used either individually or in a combination of two or more kinds with any proportions.

The proportions of the pH-dependent polymer ingredient and the hydrophobic ingredient may be decided such that the residence time in human is 12 to 24 hours, depending on the types of ingredients used, and that the gastric resident layer quickly disintegrates in the intestine. The hydrophobic ingredient is generally in the range of 0 parts by weight to 40 parts by weight, preferably 0 parts by weight to 30 parts by weight, and more preferably 0 parts by weight to 20 parts by weight, with respect to 1 part by weight of the pH-dependent polymer ingredient in the gastric resident layer.

When the pH-dependent polymer ingredient is an acidic functional group-containing polymer ingredient, the proportions of the acidic functional group-containing polymer ingredient and the hydrophobic ingredient may similarly be decided such that the residence time in human is 12 to 24 hours, depending on the types of ingredients used, and that the gastric resident layer quickly disintegrates in the intestine. The hydrophobic ingredient is generally in the range of 1 part by weight to 40 parts by weight, with respect to 1 part by weight of the acidic functional group-containing polymer ingredientin the gastric resident layer. The upper limit of the proportion is preferably at most 30 parts by weight, and more preferably at most 20 parts by weight. The lower limit of the proportion is preferably at least 2 parts by weight, and more preferably at least 5 parts by weight.

By including a water-soluble ingredient in the gastric resident layer together with the pH-dependent polymer ingredient and a necessary hydrophobic ingredient, the disintegrating property of the gastric resident layer can be controlled to adjust the gastric residence time and the ease of disintegration in the intestine. Examples of a water-soluble ingredient include: hydroxyalkyl celluloses such as hydroxypropyl cellulose, hydroxypropylmethyl cellulose (2208, 2906, 2910), and hydroxyethyl cellulose; polyvinyl derivatives such as povidone, crospovidone, and polyvinyl alcohol; polyethylene oxides; methyl cellulose; gelatin; polysaccharides such as pregelatinized starch, partially pregelatinized starch, pullulan, dextrin, sodium alginate, and gum arabic; sugars such as lactose, saccharose, trehalose, and glucose; and sugar alcohols such as mannitol, xylitol, sorbitol, erythritol, and maltitol.

The proportion of the water-soluble ingredient in the gastric resident layer may be appropriately decided such that the gastric residence time is 12 to 24 hours, and that the gastric resident layer quickly disintegrates in the intestine.

In the case where the gastric resident layer containing the enteric ingredient additionally contains a water-soluble ingredient, the proportion of the water-soluble ingredient in the gastric resident layer is preferably at most 50 % by weight, and more preferably at most 40 % by weight, because the mechanical strength of the gastric resident layer may be reduced and the gastric residence time may become insufficient when the proportion of the water-soluble ingredient in the gastric resident layer exceeds 50 % by weight.

When the gastric resident layer containing the acidic functional group-containing polymer ingredient and the hydrophobic ingredient additionally contains a water-soluble ingredient, the gastric residence time may become insufficient when the proportion of the water-soluble ingredient in the gastric resident layer exceeds 60 % by weight. For this reason, the upper limit of the proportion of the water-soluble ingredient in the gastric resident layer is preferably 60 % by weight or less, and more preferably 50 % by weight or less. On the other hand, the ease of disintegration in the intestine may suffer when the proportion of the water-soluble ingredient in the gastric resident layer is below 5 % by weight. The lower limit of the proportion of the water-soluble ingredient in the gastric resident layer is preferably 5 % by weight or more, and more preferably 10 % by weight or more.

The gastric resident layer may additionally include an excipient, a binder, a fluidizer, a lubricant, or the like. Examples of an excipient include microcrystalline cellulose, cornstarch, and anhydrous dibasic calcium phosphate. Examples of a binder include methyl cellulose, hydroxypropyl cellulose, hydroxypropylmethyl cellulose, polyvinylpyrrolidone, polyvinyl alcohol, pullulan, polyethylene glycol, gelatin, gum arabic, pregelatinized starch, and partially pregelatinized starch. Examples of a fluidizer include light anhydrous silicic acid and hydrated silicon dioxide. Examples of a lubricant include magnesium stearate, calcium stearate, polyoxyl stearate, talc, sucrose fatty acid ester, dimethylpolysiloxane, and sodium stearyl fumarate. The proportion of the ingredients above in the gastric resident layer may be appropriately decided according to the type and amount of the other ingredients used.

The gastric resident layer may be prepared by ordinary method. For example, a method may be used in which the foregoing ingredients are mixed together and the admixture is compressed to form the layer under a pressure of 300 kgf to 2000 kgf, and preferably 500 kgf to 1500 kgf.

As an ingredient for the drug releasing layer, any ingredient commonly used for orally-administered sustained-release preparations may be used. Examples of ingredients for the drug releasing layer include: microcrystalline cellulose; alkyl celluloses such as methyl cellulose and ethyl cellulose; hydroxyalkyl celluloses such as hydroxypropyl cellulose, low substituted hydroxypropyl cellulose, hydroxypropylmethyl cellulose (2208, 2906, 2910), and hydroxyethyl cellulose; carboxymethyl celluloses such as carboxymethyl cellulose, carboxymethyl cellulose calcium, and croscarmellose sodium; gums such as guar gum, xanthan gum, and gellan gum; polyethylene oxides; aminoalkylmethacrylate copolymers; acrylic acid copolymers; carboxyvinyl polymers; polyvinyl pyrroridones; polyvinyl alcohols; macrogols; oils and fats such as carnauba wax and hydrogenated oil; starches such as cornstarch, potato starch, wheat starch, dextrin, pregelatinized starch, partially pregelatinized starch, sodium carboxymethyl starch, and pullulan; sugars such as lactose and saccharose; sugar alcohols such as mannitol, xylitol, sorbitol, and maltitol; minerals such as kaolin, talc, magnesium stearate, titanium oxide, precipitated calcium carbonate, and calcium hydrogen phosphate; and plasticizers such as triethyl citrate, propylene glycol, triacetin, and medium-chain triglyceride. Desired sustained-release conditions can be set by appropriately selecting these ingredients. Among these ingredients, it is preferable to include hydroxyalkyl cellulose or polyethylene oxide. The ingredient may be used either individually or in a combination of two or more kinds with any proportions.

Drugs contained in the drug releasing layer may be any drug that requires sustained release. Examples of such drugs include: drugs that are absorbed in the duodenum and/or the jejunum; drugs that exhibit their effect in the stomach and/or the upper small intestine; drugs that are decomposed in the lower digestive tract; drugs that are susceptible to efflux into the digestive tract by drug transporters present in the intestinal cells in the lower digestive tract; and drugs that are insolubilized in an alkaline or neutral environment in the lower digestive tract.

Examples of drugs that are absorbed in the duodenum and/or the jejunum include: a sodium-dependent glucose transporter inhibitor; a diuretic such as fluosemide; a blood coagulation factor Xa inhibitor; an immunosuppressants such as ciclosporin; and a β receptor agonist.

Examples of drugs that exhibit their effect in the stomach and/or in the upper small intestine include: sodium-dependent glucose transporter 1 inhibitors; carbohydrase inhibitors such as voglibose, acarbose, and miglitol; purine absorption inhibitors; urease inhibitors; antibacterial agents; antitumor drugs such as a proton pump inhibitor, an H₂ blocker, and a mucosa-protecting antitumor drug; antacids; acid secretion inhibitors; and drugs for controlling intestinal function.

Examples of drugs that are decomposed in the lower digestive tract include: agents affecting the central nervous system, agents affecting circulatory organs, agents affecting respiratory organs, agents affecting digestive organs, hormonal agents, antihistamines, metabolic drugs, antitumor drugs, antibiotics, and chemotherapeutic drugs.

Examples of drugs that are susceptible to efflux into the digestive tract by drug transporters present in the intestinal cells in the lower digestive tract include: anticancer agents such as paclitaxel; antibacterial agents such as ciprofloxacin; and HIV protease inhibitors such as saquinavir, ritonavir, and nelfinavir.

Examples of drugs that are insolubilized in an alkaline or neutral environment in the lower digestive tract include: iron salts; digoxin; and antifungal agents such as ketoconazole, fluconazole, griseofulvin, itraconazole, and mitoconazole.

The drug releasing layer may be prepared by ordinary method. For example, a method may be used in which the foregoing ingredient and drug are mixed together and the admixture is compressed to form the layer under a pressure of 300 kgf to 2000 kgf, and preferably 500 kgf to 1500 kgf.

A gastric retentive preparation of the present invention is a bilayer or multilayer tablet including the gastric resident layer and the drug releasing layer, and may be prepared by methods commonly used for the preparation of bilayer or multilayer tablets. For example, the gastric retentive preparation may be prepared by a method in which the ingredients respectively used for the gastric resident layer and the drug releasing layer are separately loaded into a tableting machine, and are compressed into a bilayer or multilayer tablet under a pressure of 300 kgf to 2000 kgf, and preferably 500 kgf to 1500 kgf. The gastric retentive preparation may also be prepared, for example, by a method in which the gastric resident layer and the drug releasing layer are bonded together after being prepared by the foregoing methods. The bonding of the gastric resident layer and the drug releasing layer may be made, for example, by tablet compression of the gastric resident layer and the drug releasing layer, or by tablet compression using a bonding layer between the gastric resident layer and the drug releasing layer, the bonding layer including a binder such as hydroxypropyl cellulose.

The gastric resident layer including the enteric ingredient, particularly an acrylic acid copolymer are relatively hydrophobic, whereas the drug releasing layer including hydroxyalkyl cellulose or polyethylene oxide is relatively hydrophilic. For this reason, desirable bonding may not achieved by the simple tablet compression of the two materials. It is therefore preferable, when bonding is made, to include the water-soluble ingredient in the gastric resident layer and thereby improve the strength of bonding.

The gastric retentive preparation may have any shape as long as it is suited for manufacture, ingestion, or the like. Some of the possible shapes are lens, disc, circular, caplet, oval, triangle, quadrangle, pentagonal, hexagonal, flower, and elliptical.

Preferably, the gastric retentive preparation is sized so that the lower limit of its diameter (short axis in the case of shaped tablet) is greater than 6 mm, and particularly 7 mm or greater. A sufficient gastric residence time may not be obtained when the diameter is 6 mm or less. The upper limit of diameter (long axis in the case of shaped tablet) may be any length provided that it permits ingestion. Generally, the upper limit of diameter is 30 mm or less.

For ease of identification, the gastric retentive preparation may include a colorant, such as a dye, in the drug releasing layer and/or the gastric resident layer. Any colorant may be used as long as it is usable for medicines. Some of the examples include: food dyes such as food yellow 5, food red 2, and food blue 2; food lake dyes; ferric oxide; and yellow ferric oxide.

### EXAMPLES

The following will describe the present invention in more detail based on Examples. The present invention is not limited in any ways by the following Examples.

### (Dissolution Test)

According to the paddle method described in the JP dissolution testing method, a test preparation was placed in a sinker and was immersed in the JP first fluid (900 mL), which was then stirred for 15 hours at 37°C at a paddle speed of 200 rpm. The test preparation was gently taken out and was immersed in the JP second fluid (900 mL). After stirring the solution for 9 hours at 37°C at a paddle speed of 200 rpm, the large diameter of the gastric resident layer of the preparation was measured with a caliper. In the case where no remains of the gastric resident layer were observed, or when the remains of the gastric resident layer appeared spongy or only a small portion of the soft material was present, the time from the start of stirring was recorded as the dissolution time.

### (Strength Test)

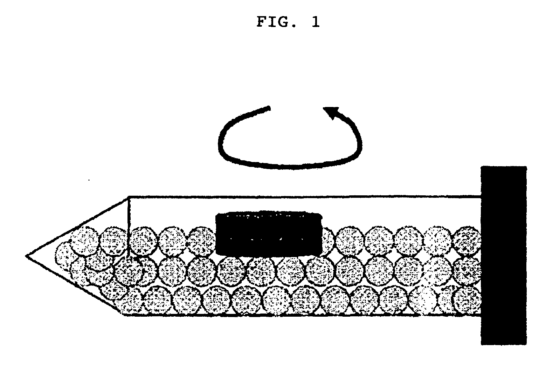

A 50 mL volume Falcon^{™} conical tube (30x115 mm) for centrifugation (BlueMax; Becton, Dickinson and Company, Japan) was charged with one tablet of test preparation, 50 g of glass beads BZ-4 (AS ONE Corporation) having an outer diameter of 3.962 mm to 4.699 mm, and 30 mL of the JP first fluid. The tube was horizontally laid and was rotated at 200 rpm at 37°C for 10 hours with the JP first fluid (30 mL) replaced every 2.5 hours. Thereafter, the small diameter of the gastric resident layer of the preparation was measured with a caliper. The test preparation thus obtained was then placed on a rheometer (SUN RHEO METER CR-150; SUN SCIENTIFIC CO., LTD.), with the gastric resident layer on top and the drug releasing layer at the bottom. A maximum load was measured at a compression clearance of 1 mm and a measurement range of 10 kg, using a 3.5 cm flat circular compression adapter. Fig. 1 illustrates the apparatus used for the test.

### (Gastric Residence Time)

The test preparation was orally administered to groups of 2 to 3 beagles, 8 to 10 months of age, and the dogs were x-rayed over a time course to measure gastric residence time. Specifically, each group of beagles was put in a cage with free access to water. The dogs were fasted for at least 20 hours, and were given a mixture of 250 g of solid dog food and 50 g of chum 30 minutes before administration of the preparation. Upon confirmation that all the food had been eaten, the test preparation was administered together with 20 mL of water. This was followed by administration of 20 w/v% barium sulfate (10 mL/body), and the dogs were deprived of water until the end of test. After administration of the test preparation, the subjects were x-rayed at one hour intervals, and 20 w/v% barium sulfate (10 mL/body) was administered as appropriate during the course of x-ray radiography. Note that, the test preparation was embedded with a circular tablet for x-ray imaging at each center of the drug releasing layer and the gastric resident layer, which tablet had been prepared from a mixture of barium sulfate (500 mg) and hydroxypropyl cellulose (HPC-H fine powder, 500 mg) using an IR-measurement tableting machine measuring 3 mm in diameter. The gastric residence time was given by the mean value of the time period from the administration of the test preparation to the last observation of the preparation in the stomach by x-ray radiography. For cases the preparation was observed in the stomach 16 hours after the administration but was not observed 24 hours after the administration, the gastric residence time was deemed as 16 hours.

### (Example 1)

Furosemide (1250 mg), hydroxypropylmethyl cellulose 2208 (Metolose 90SH4000SR, 3250 mg), and hydroxypropyl cellulose (HPC-M fine powder, 500 mg) were mixed together in a mortar to prepare a composition for the drug releasing layer. Separately, hydroxypropylmethyl cellulose acetate succinate (Shin-Etsu AQOAT AS-MF, 800 mg), hydroxypropyl cellulose (HPC-M fine powder, 200 mg), and yellow ferric oxide (5 mg) were mixed together in a mortar to prepare a composition for the gastric resident layer. The composition for the drug releasing layer and the composition for the gastric resident layer were loaded into a tableting machine (N-30E, OKADA SEIKO CO., LTD.) in this order, and the compositions were compressed into a tablet at a punch pressure of about 1000 kgf/cm², using a circular die and punch measuring 8 mm in diameter. As a result, flat circular tablets of gastric retentive preparation having a diameter of 8 mm were prepared, each containing: furosemide (50 mg), hydroxypropylmethyl cellulose 2208 (130 mg), and hydroxypropyl cellulose (20 mg) for the drug releasing layer; and hydroxypropylmethyl cellulose acetate succinate (80 mg), hydroxypropyl cellulose (20 mg), and yellow ferric oxide (0.5 mg) for the gastric resident layer. Note that, when loading the composition for the drug releasing layer, the tablet for x-ray imaging was placed at the center of the composition after half of the composition had been loaded, and the rest of the composition was loaded thereon to embed the tablet in the drug releasing layer. In the same manner, when loading the composition for the gastric resident layer, the tablet for x-ray imaging was placed at the center of the composition after half of the composition had been loaded, and the rest of the composition was loaded thereon to embed the tablet in the gastric resident layer.

### (Dissolution Test)

Small diameter (JP first fluid): 8.53 mm
Dissolution time (JP second fluid): 3.0 hours
(Strength Test)
Small diameter (JP first fluid) : 8.32 mm, maximum load: > 10000 g
(Confirmation Test for Gastric Residence Time)
Gastric residence time: 16 hours

### (Example 2)

According to Example 1 , flat circular tablets of gastric retentive preparation having a diameter of 8 mm were prepared, each containing: furosemide (50 mg), hydroxypropylmethyl cellulose 2208 (Metolose 90SH4000SR, 130 mg), and hydroxypropyl cellulose (20 mg) for the drug releasing layer; and hydroxypropylmethyl cellulose acetate succinate (70 mg), lactose (30 mg), and yellow ferric oxide (0.5 mg) for the gastric resident layer.

### (Dissolution Test)

Small diameter (JP first fluid): 8.42 mm
Dissolution time (JP second fluid): 1.0 hour
(Strength Test)
Small diameter (JP first fluid) : 8.23 mm, maximum load: > 10000 g
(Confirmation Test for Gastric Residence Time)
Gastric residence time: 16 hours

### (Example 3)

According to Example 1, flat circular tablets of gastric retentive preparation having a diameter of 8 mm were prepared, each containing: furosemide (50 mg), hydroxypropylmethyl cellulose 2208 (Metolose 90SH4000SR, 130 mg), and hydroxypropyl cellulose (20 mg) for the drug releasing layer; and methacrylic acid-ethylacrylate copolymer (Eudragit L100-55, 80 mg), hydroxypropyl cellulose (20 mg), and yellow ferric oxide (0.5 mg) for the gastric resident layer.

### (Dissolution Test)

Small diameter (JP first fluid): 9.32 mm
Dissolution time (JP second fluid): 3.0 hours
(Strength Test)
Small diameter (JP first fluid) : 9.23 mm, maximum load: > 10000 g
(Confirmation Test for Gastric Residence Time)
Gastric residence time: 16 hours

### (Example 4)

According to Example 1, flat circular tablets of gastric retentive preparation having a diameter of 8 mm were prepared, each containing: furosemide (50 mg), hydroxypropylmethyl cellulose 2208 (Metolose 90SH4000SR, 130 mg), and hydroxypropyl cellulose (20 mg) for the drug releasing layer; and methacrylic acid-ethylacrylate copolymer (80 mg), and hydroxypropylmethyl cellulose 2208 (Metolose 90SH100SR, 20 mg) for the gastric resident layer.

(Dissolution Test)
Small diameter (JP first fluid): 9.84 mm
Dissolution time (JP second fluid): 5.0 hours
(Strength Test)
Small diameter (JP first fluid) : 9.31 mm, maximum load: > 10000 g

### (Example 5)

According to Example 1, flat circular tablets of gastric retentive preparation having a diameter of 8 mm were prepared, each containing: furosemide (50 mg), hydroxypropylmethyl cellulose 2208 (Metolose 90SH4000SR, 130 mg), and hydroxypropyl cellulose (20 mg) for the drug releasing layer; and methacrylic acid-ethylacrylate copolymer (80 mg), and hydroxypropylmethyl cellulose 2208(Metolose 90SH4000SR, 20 mg) for the gastric resident layer.

(Dissolution Test)
Small diameter (JP first fluid): 10.29 mm
Large diameter (JP second fluid): 1.71 mm
(Strength Test)
Small diameter (JP first fluid): 9.41 mm, maximum load: > 10000 g

### (Example 6)

According to Example 1, flat circular tablets of gastric retentive preparation having a diameter of 8 mm were prepared, each containing: furosemide (50 mg), hydroxypropylmethyl cellulose 2208 (Metolose 90SH4000SR, 130 mg), and hydroxypropyl cellulose (20 mg) for the drug releasing layer; and methacrylic acid-ethylacrylate copolymer (80 mg), and hydroxypropylmethyl cellulose 2208(Metolose 90SH100000SR, 20 mg) for the gastric resident layer.

(Strength Test)
Small diameter (JP first fluid) : 9.65 mm, maximum load: > 10000 9

### (Example 7)

According to Example 1, flat circular tablets of gastric retentive preparation having a diameter of 8 mm were prepared, each containing: furosemide (50 mg), hydroxypropylmethyl cellulose 2208 (Metolose 90SH4000SR, 130 mg), and hydroxypropyl cellulose (20 mg) for the drug releasing layer; and methacrylic acid-ethylacrylate copolymer (70 mg), hydroxypropylmethyl cellulose 2208(Metolose 90SH4000SR, 20 mg), and lactose (10 mg) for the gastric resident layer.

(Dissolution Test)
Small diameter (JP first fluid): 10.25 mm
Dissolution time (JP second fluid): 7.0 hours
(Strength Test)
Small diameter (JP first fluid) : 9.62 mm, maximum load: 9760 g
(Confirmation Test for Gastric Residence Time)
Gastric residence time: 19 hours

### (Example 8)

According to Example 1, flat circular tablets of gastric retentive preparation having a diameter of 8 mm were prepared, each containing: furosemide (50 mg), hydroxypropylmethyl cellulose 2208 (Metolose 90SH4000SR, 130 mg), and hydroxypropyl cellulose (20 mg) for the drug releasing layer; and methacrylic acid-ethylacrylate copolymer (60 mg), and hydroxypropylmethyl cellulose 2208(Metolose 90SH4000SR, 40 mg) for the gastric resident layer.

(Strength Test)
Small diameter (JP first fluid) : 9.34 mm, maximum load: 6490 g

### (Example 9)

According to Example 1, flat circular tablets of gastric retentive preparation having a diameter of 8 mm were prepared, each containing: furosemide (50 mg), hydroxypropylmethyl cellulose 2208 (Metolose 90SH4000SR, 130 mg), and hydroxypropyl cellulose (20 mg) for the drug releasing layer; and methacrylic acid-ethylacrylate copolymer (70 mg), and hydroxypropylmethyl cellulose 2208(Metolose 90SH4000SR, 30 mg) for the gastric resident layer.

(Dissolution Test)
Small diameter (JP first fluid): 10.43 mm
Large diameter (JP second fluid): 1.82 mm
(Strength Test)
Small diameter (JP first fluid) : 9.90 mm, maximum load: > 10000 g

### (Example 10)

According to Example 1, flat circular tablets of gastric retentive preparation having a diameter of 8 mm were prepared, each containing: furosemide (50 mg), hydroxypropylmethyl cellulose 2208 (Metolose 90SH4000SR, 130 mg), and hydroxypropyl cellulose (20 mg) for the drug releasing layer; and methacrylic acid-ethylacrylate copolymer (40 mg) and hydroxypropyl cellulose (10 mg) for the gastric resident layer.

(Dissolution Test)
Small diameter (JP first fluid): 8.74 mm
Dissolution time (JP second fluid): 3.5 hours
(Strength Test)
Small diameter (JP first fluid): 8.79 mm, maximum load: 6130 g

### (Example 11)

According to Example 1, flat circular tablets of gastric retentive preparation having a diameter of 8 mm were prepared, each containing: ciprofloxacin (30 mg), polyethylene oxide (POLYOX WSR-303, 85 mg), and hydroxypropyl cellulose (85 mg) for the drug releasing layer; and methacrylic acid-ethylacrylate copolymer (35 mg), hydroxypropylmethyl cellulose 2208 (Metolose 90SH100SR, 10 mg) , and low substituted hydroxypropyl cellulose (5 mg) for the gastric resident layer.

(Dissolution Test)
Small diameter (JP first fluid): 9.65 mm
Dissolution time (JP second fluid): 3.0 hours
(Strength Test)
Small diameter (JP first fluid): 9.21 mm, maximum load: 5140 g
(Confirmation Test for Gastric Residence Time)
Gastric residence time: 15 hours

### (Example 12)

According to Example 1, flat circular tablets of gastric retentive preparation having a diameter of 8 mm were prepared, each containing: furosemide (50 mg), hydroxypropylmethyl cellulose 2208 (Metolose 90SH4000SR, 130 mg), and hydroxypropyl cellulose (20 mg) for the drug releasing layer; and hydrogenated oil (75 mg), carboxyvinylpolymer (5 mg), polyethylene oxide (5 mg), and anhydrous dibasic calcium phosphate (15 mg) for the gastric resident layer.

(Dissolution Test)
Small diameter (JP first fluid): 8.00 mm
Dissolution time (JP second fluid): 8.0 hours
(Strength Test)
Small diameter (JP first fluid) : 7.00 mm, maximum load: 6720 g
(Confirmation Test for Gastric Residence Time)
Gastric residence time: 19 hours

### (Example 13)

According to Example 1, flat circular tablets of gastric retentive preparation having a diameter of 8 mm were prepared, each containing: furosemide (50 mg), hydroxypropylmethyl cellulose 2208 (Metolose 90SH4000SR, 130 mg), and hydroxypropyl cellulose (20 mg) for the drug releasing layer; and ethyl cellulose (Ethocel STD10FP, 80 mg), carboxyvinylpolymer (10 mg), and polyethylene oxide (POLYOX WSR 301, 10 mg) for the gastric resident layer.

(Dissolution Test)
Small diameter (JP first fluid): 8.82 mm
Large diameter (JP second fluid): 4.14 mm
(Strength Test)
Small diameter (JP first fluid) : 8.47 mm, maximum load: 7750 g
(Confirmation Test for Gastric Residence Time)
Gastric residence time: 16 hours

### (Example 14)

According to Example 1 except for using a shaped tablet die and punch having a large diameter of 14 mm and a small diameter of 7 mm, shaped tablets of gastric retentive preparation having a large diameter of 14 mm and a small diameter of 7 mm were prepared, each containing: furosemide (50 mg), hydroxypropylmethyl cellulose 2208 (Metolose 90SH4000SR, 150 mg), hydroxypropylmethyl cellulose 2910 (Metolose 60SH50 , 115 mg), and hydroxypropyl cellulose (35 mg) for the drug releasing layer; and hydrogenated oil (135 mg), carboxyvinylpolymer (7.5 mg), and crospovidone (Polyplasdone XL-10, 7.5 mg) for the gastric resident layer.

(Dissolution Test)
Small diameter (JP first fluid): 7.14 mm
Dissolution time (JP second fluid): 8 hours
(Strength Test)
Small diameter (JP first fluid): 7.05 mm, maximum load: > 10000 g
(Confirmation Test for Gastric Residence Time)
Gastric residence time: 19 hours

### (Example 15)

According to Example 1 except for using a shaped tablet die and punch having a large diameter of 17.5 mm and a small diameter of 7.5 mm, shaped tablets of gastric retentive preparation having a large diameter of 17.5 mm and a small diameter of 7.5 mm were prepared, each containing: furosemide (50 mg), hydroxypropylmethyl cellulose 2208 (Metolose 90SH4000SR, 150 mg), hydroxypropylmethyl cellulose 2910 (Metolose 60SH50, 205 mg), and hydroxypropyl cellulose (45 mg) for the drug releasing layer; and methacrylic acid-ethylacrylate copolymer (140 mg), hydroxypropyl cellulose (40 mg), and lactose (20 mg) for the gastric resident layer.

(Dissolution Test)
Small diameter (JP first fluid): 8.25 mm
Dissolution time (JP second fluid): 7 hours
(Strength Test)
Small diameter (JP first fluid) : 7.82 mm, maximum load: > 10000 9
(Confirmation Test for Gastric Residence Time)
Gastric residence time: 16 hours

### (Example 16)

According to Example 1 except for using a shaped tablet die and punch having a large diameter of 17.5 mm and a small diameter of 7.5 mm, shaped tablets of gastric retentive preparation having a large diameter of 17.5 mm and a small diameter of 7.5 mm were prepared, each containing: furosemide (50 mg), hydroxypropylmethyl cellulose 2208 (Metolose 90SH4000SR, 150 mg), hydroxypropylmethyl cellulose 2910 (Metolose 60SH50, 205 mg), and hydroxypropyl cellulose (45 mg) for the drug releasing layer; and methacrylic acid-ethylacrylate copolymer (160 mg) and hydroxypropylmethyl cellulose 2208 (Metolose 90SH100SR, 40 mg) for the gastric resident layer.

(Dissolution Test)
Small diameter (JP first fluid): 8.98 mm
Dissolution time (JP second fluid): 4 hours
(Strength Test)
Small diameter (JP first fluid) : 8.55 mm, maximum load: > 10000 g
(Confirmation Test for Gastric Residence Time)
Gastric residence time: 19 hours

### (Example 17)

vAccording to Example 1 except for using a shaped tablet die and punch having a large diameter of 17.5 mm and a small diameter of 7.5 mm, shaped tablets of gastric retentive preparation having a large diameter of 17.5 mm and a small diameter of 7.5 mm were prepared, each containing: furosemide (50 mg), hydroxypropylmethyl cellulose 2208 (Metolose 90SH4000SR, 150 mg), hydroxypropylmethyl cellulose 2910 (Metolose 60SH50, 205 mg), and hydroxypropyl cellulose (45 mg) for the drug releasing layer; and hydroxypropylmethyl cellulose acetate succinate (160 mg), and hydroxypropylmethyl cellulose 2208(Metolose 90SH100SR, 40 mg) for the gastric resident layer.

(Dissolution Test)
Small diameter (JP first fluid): 8.76 mm
Dissolution time (JP second fluid): 4 hours
(Strength Test)
Small diameter (JP first fluid) : 8.43 mm, maximum load: > 10000 g
(Confirmation Test for Gastric Residence Time)
Gastric residence time: 17 hours

### (Example 18)

According to Example 1 except for using a shaped tablet die and punch having a large diameter of 14 mm and a small diameter of 7 mm, shaped tablets of gastric retentive preparation having a large diameter of 14 mm and a small diameter of 7 mm were prepared, each containing: furosemide (50 mg), hydroxypropylmethyl cellulose 2208 (Metolose 90SH4000SR, 150 mg), hydroxypropylmethyl cellulose 2910 (Metolose 60SH50, 115 mg), and hydroxypropyl cellulose (35 mg) for the drug releasing layer; and methacrylic acid-ethylacrylate copolymer (52.5 mg), hydroxypropylmethyl cellulose acetate succinate (52.5 mg), hydroxypropylmethyl cellulose 2208(Metolose 90SH100SR, 30 mg), and lactose (15 mg) for the gastric resident layer.

(Dissolution Test)
Small diameter (JP first fluid): 8.79 mm
Dissolution time (JP second fluid): 5 hours
(Strength Test)
Small diameter (JP first fluid): 8.35 mm, maximum load: 7940 g
(Confirmation Test for Gastric Residence Time)
Gastric residence time: 18 hours

### (Example 19)

According to Example 1 except for using a shaped tablet die and punch having a large diameter of 17.5 mm and a small diameter of 7.5 mm, shaped tablets of gastric retentive preparation having a large diameter of 17.5 mm and a small diameter of 7.5 mm were prepared, each containing: furosemide (50 mg), hydroxypropylmethyl cellulose 2208 (Metolose 90SH4000SR, 150 mg), hydroxypropylmethyl cellulose 2910 (Metolose 60SH50, 205 mg), and hydroxypropyl cellulose (45 mg) for the drug releasing layer; and hydroxypropylmethyl cellulose acetate succinate (160 mg), and hydroxypropyl cellulose (40 mg) for the gastric resident layer.

(Dissolution Test)
Small diameter (JP first fluid): 8.21 mm
Dissolution time (JP second fluid): 4 hours
(Strength Test)
Small diameter (JP first fluid) : 7.97 mm, maximum load: > 10000 g

### (Example 20)

According to Example 1, flat circular tablets of gastric retentive preparation having a diameter of 8 mm were prepared, each containing: furosemide (50 mg), hydroxypropylmethyl cellulose 2208 (Metolose 90SH4000SR, 130 mg), and hydroxypropyl cellulose (20 mg) for the drug releasing layer; and hydroxypropylmethyl cellulose acetate succinate (100 mg)for the gastric resident layer.

(Dissolution Test)
Small diameter (JP first fluid): 8.09 mm
Dissolution time (JP second fluid): 2.5 hours
(Strength Test)
Small diameter (JP first fluid): 8.00 mm, maximum load: > 10000 g

### (Example 21)

According to Example 1, flat circular tablets of gastric retentive preparation having a diameter of 8 mm were prepared, each containing: furosemide (50 mg), hydroxypropylmethyl cellulose 2208 (Metolose 90SH4000SR, 130 mg), and hydroxypropyl cellulose (20 mg) for the drug releasing layer; and hydroxypropylmethyl cellulose acetate succinate (70 mg) and hydroxypropyl cellulose (30 mg) for the gastric resident layer.

(Dissolution Test)
Small diameter (JP first fluid): 9.00 mm
Dissolution time (JP second fluid): 8 hours
(Strength Test)
Small diameter (JP first fluid): 8.51 mm, maximum load: 6770 g

### (Example 22)

According to Example 1, flat circular tablets of gastric retentive preparation having a diameter of 8 mm were prepared, each containing: furosemide (50 mg), hydroxypropylmethyl cellulose 2208 (Metolose 90SH4000SR, 130 mg), and hydroxypropyl cellulose (20 mg) for the drug releasing layer; and hydroxypropylmethyl cellulose acetate succinate (60 mg), hydroxypropyl cellulose (5 mg), and lactose (35 mg) for the gastric resident layer.

(Dissolution Test)
Small diameter (JP first fluid): 8.38 mm
Dissolution time (JP second fluid): 2 hours
(Strength Test)
Small diameter (JP first fluid) : 8.20 mm, maximum load: 8460 g

### (Example 23)

According to Example 1, flat circular tablets of gastric retentive preparation having a diameter of 8 mm were prepared, each containing: furosemide (50 mg), hydroxypropylmethyl cellulose 2208 (Metolose 90SH4000SR, 130 mg), and hydroxypropyl cellulose (20 mg) for the drug releasing layer; and methacrylic acid-ethylacrylate copolymer (40 mg) and hydroxypropylmethyl cellulose 2208(Metolose 90SH100SR, 10 mg) for the gastric resident layer.

(Dissolution Test)
Small diameter (JP first fluid): 9.34 mm
Dissolution time (JP second fluid): 2 hours
(Strength Test)
Small diameter (JP first fluid): 9.35 mm, maximum load: 6790 g

### (Example 24)

According to Example 1, flat circular tablets of gastric retentive preparation having a diameter of 8 mm were prepared, each containing: ciprofloxacin (30 mg), polyethylene oxide (POLYOX WSR-303, 85 mg), and hydroxypropyl cellulose (85 mg) for the drug releasing layer; and methacrylic acid-ethylacrylate copolymer (35 mg), hydroxypropylmethyl cellulose 2208(Metolose 90SH100SR, 10 mg), and carmellose calcium (5 mg) for the gastric resident layer.

(Dissolution Test)
Small diameter (JP first fluid): 9.59 mm
Dissolution time (JP second fluid): 3 hours
(Strength Test)
Small diameter (JP first fluid): 8.96 mm, maximum load: 5120 g

### (Example 25)

According to Example 1, flat circular tablets of gastric retentive preparation having a diameter of 8 mm were prepared, each containing: furosemide (50 mg), hydroxypropylmethyl cellulose 2208 (Metolose 90SH4000SR, 130 mg), and hydroxypropyl cellulose (20 mg) for the drug releasing layer; and milled hydroxypropylmethyl cellulose phthalate 200731 (HPMCPHP-55, 80 mg), and hydroxypropyl cellulose (20 mg) for the gastric resident layer.

(Dissolution Test)
Small diameter (JP first fluid): 8.34 mm
Dissolution time (JP second fluid): 5 hours
(Strength Test)
Small diameter (JP first fluid) : 8.14 mm, maximum load: > 10000 g

### (Example 26)

According to Example 1, flat circular tablets of gastric retentive preparation having a diameter of 8 mm were prepared, each containing: furosemide (50 mg), hydroxypropylmethyl cellulose 2208 (Metolose 90SH4000SR, 130 mg), and hydroxypropyl cellulose (20 mg) for the drug releasing layer; and milled cellulose acetate phthalate (80 mg) and hydroxypropyl cellulose (20 mg) for the gastric resident layer.

(Dissolution Test)
Small diameter (JP first fluid): 8.31 mm
Dissolution time (JP second fluid): 2.5 hours
(Strength Test)
Small diameter (JP first fluid): 7.82 mm, maximum load: > 10000 g

### (Example 27)

According to Example 1, flat circular tablets of gastric retentive preparation having a diameter of 8 mm were prepared, each containing: ciprofloxacin (30 mg), polyethylene oxide (POLYOX WSR-303, 85 mg), and hydroxypropyl cellulose (85 mg) for the drug releasing layer; and hydrogenated oil (90 mg), polyethylene oxide (POLYOX WSR N-750, 5 mg), and carmellose calcium (ECG-505, 5 mg) for the gastric resident layer.

(Dissolution Test)
Small diameter (JP first fluid): 8.03 mm
Dissolution time (JP second fluid): 5.34 mm
(Strength Test)
Small diameter (JP first fluid): 8.12 mm, maximum load: > 10000 g
(Confirmation Test for Gastric Residence Time)
Gastric residence time: 14 hours

### (Example 28)

According to Example 1, flat circular tablets of gastric retentive preparation having a diameter of 8 mm were prepared, each containing: metformin (50 mg), hydroxypropylmethyl cellulose 2208 (Metolose 90SH30000F, 130 mg), and hydroxypropyl cellulose (20 mg) for the drug releasing layer; and hydrogenated oil (80 mg), carboxyvinylpolymer (10 mg), and polyethylene oxide (POLYOX WSR 301, 10 mg) for the gastric resident layer.

(Dissolution Test)
Small diameter (JP first fluid): 9.00 mm
Dissolution time (JP second fluid): 9 hours
(Strength Test)
Small diameter (JP first fluid): 8.80 mm, maximum load: 5160 g

### (Example 29)

According to Example 1, flat circular tablets of gastric retentive preparation having a diameter of 8 mm were prepared, each containing: furosemide (50 mg), hydroxypropylmethyl cellulose 2208 (Metolose 90SH4000SR, 130 mg), and hydroxypropyl cellulose (20 mg) for the drug releasing layer; and hydrogenated oil (70 mg), carboxyvinylpolymer (5 mg), and polyethylene oxide (POLYOX WSR Coagulant, 5 mg), and anhydrous dibasic calcium phosphate (20 mg) for the gastric resident layer.

(Dissolution Test)
Small diameter (JP first fluid): 8.27 mm
Dissolution time (JP second fluid): 4.85 mm
(Strength Test)
Small diameter (JP first fluid) : 7.70 mm, maximum load: 5300 g

### (Example 30)

According to Example 1, flat circular tablets of gastric retentive preparation having a diameter of 8 mm were prepared, each containing: furosemide (50 mg), hydroxypropylmethyl cellulose 2208 (Metolose 90SH4000SR, 130 mg), and hydroxypropyl cellulose (20 mg) for the drug releasing layer; and hydrogenated oil (75 mg), carboxyvinylpolymer (10 mg), and polyethylene oxide (POLYOX WSR Coagulant, 10 mg), and microcrystalline cellulose (Avicel PH101, 5 mg) for the gastric resident layer.

(Dissolution Test)
Small diameter (JP first fluid): 9.21 mm
Dissolution time (JP second fluid): 5.79 mm
(Strength Test)
Small diameter (JP first fluid) : 8.70 mm, maximum load: 5180 g

### (Example 31)

According to Example 1, flat circular tablets of gastric retentive preparation having a diameter of 8 mm were prepared, each containing: furosemide (50 mg), hydroxypropylmethyl cellulose 2208 (Metolose 90SH4000SR, 130 mg), and hydroxypropyl cellulose (20 mg) for the drug releasing layer; and hydrogenated oil (80 mg), carboxyvinylpolymer (5 mg), polyethylene oxide (POLYOX WSR Coagulant, 5 mg), and anhydrous dibasic calcium phosphate (10 mg) for the gastric resident layer.

(Dissolution Test)
Small diameter (JP first fluid): 8.27 mm
Dissolution time (JP second fluid): 4.84 mm
(Strength Test)
Small diameter (JP first fluid): 8.01 mm, maximum load: 8090 g

### (Example 32)

According to Example 1, flat circular tablets of gastric retentive preparation having a diameter of 8 mm were prepared, each containing: furosemide (50 mg), hydroxypropylmethyl cellulose 2208 (Metolose 90SH4000SR, 130 mg), and hydroxypropyl cellulose (20 mg) for the drug releasing layer; and hydrogenated oil (90 mg), carboxyvinylpolymer (5 mg), and polyethylene oxide (POLYOX WSR-N750, 5 mg) for the gastric resident layer.

(Dissolution Test)
Small diameter (JP first fluid): 9.21 mm
Dissolution time (JP second fluid): 4.13 mm
(Strength Test)
Small diameter (JP first fluid): 8.10 mm, maximum load: > 10000 g

### (Example 33)

According to Example 1, flat circular tablets of gastric retentive preparation having a diameter of 8 mm were prepared, each containing: furosemide (50 mg), hydroxypropylmethyl cellulose 2208 (Metolose 90SH4000SR, 130 mg), and hydroxypropyl cellulose (20 mg) for the drug releasing layer; and hydrogenated oil (90 mg) and carboxyvinylpolymer (10 mg) for the gastric resident layer.

(Dissolution Test)
Small diameter (JP first fluid): 8.41 mm
Dissolution time (JP second fluid): 4.77 mm
(Strength Test)
Small diameter (JP first fluid) : 8.26 mm, maximum load: > 10000 g

### (Example 34)

According to Example 1, flat circular tablets of gastric retentive preparation having a diameter of 8 mm were prepared, each containing: furosemide (50 mg), hydroxypropylmethyl cellulose 2208 (Metolose 90SH4000SR, 130 mg), and hydroxypropyl cellulose (20 mg) for the drug releasing layer; and hydrogenated oil (90 mg), polyethylene oxide (POLYOX WSR-N750, 5 mg), and sodium carboxymethyl starch (Primojel, 5 mg) for the gastric resident layer.

(Dissolution Test)
Small diameter (JP first fluid): 8.08 mm
Dissolution time (JP second fluid): 5.53 mm
(Strength Test)
Small diameter (JP first fluid) : 8.02 mm, maximum load: > 10000 g

### (Example 35)

According to Example 1, flat circular tablets of gastric retentive preparation having a diameter of 8 mm were prepared, each containing: furosemide (50 mg), hydroxypropylmethyl cellulose 2208 (Metolose 90SH4000SR, 130 mg), and hydroxypropyl cellulose (20 mg) for the drug releasing layer; and hydrogenated oil (90 mg), carboxyvinylpolymer (5 mg), and povidone (PLASDONE K29/32, 5 mg) for the gastric resident layer.

(Dissolution Test)
Small diameter (JP first fluid): 8.00 mm
Dissolution time (JP second fluid): 4.97 mm
(Strength Test)
Small diameter (JP first fluid): 8.05 mm, maximum load: > 10000 g

### (Comparative Example 1)

According to Example 1 except for using a shaped tablet die and punch having a large diameter of 17.5 mm and a small diameter of 7.5 mm, shaped tablets of gastric retentive preparation having a large diameter of 17.5 mm and a small diameter of 7.5 mm were prepared, each containing: furosemide (50 mg), hydroxypropylmethyl cellulose 2208 (Metolose 90SH4000SR, 150 mg), hydroxypropylmethyl cellulose 2910 (Metolose 60SH50, 205 mg), and hydroxypropyl cellulose (45 mg) for the drug releasing layer; and xanthan gum (180 mg), hydroxypropylmethyl cellulose 2208(Metolose 90SH30000F, 157.5 mg), microcrystalline cellulose (76.5 mg), and guar gum (36 mg) for the gastric resident layer.

(Dissolution Test)
Small diameter (JP first fluid): 9.30 mm
Large diameter (JP second fluid): 21.74 mm
(Strength Test)
Small diameter (JP first fluid) : 7.59 mm, maximum load: 4520 g
(Confirmation Test for Gastric Residence Time)
Gastric residence time: 8 hours

Separately, the preparation was orally administered to beagles. The abdomen was opened 8 hours after administration, and the preparation remaining in the stomach was taken out to measure the small diameter of the gastric resident layer and maximum load of the preparation (*in vivo* strength test). Note that, the preparation was also treated in JP first fluid at 37°C for 8 hours to measure the small diameter and maximum load of the preparation according to the strength test described above (*in vitro* strength test).
(*In Vivo* Strength Test)
Small diameter: 7.18 mm, maximum load: 6370 g
(*In Vitro* Strength Test)
Small diameter: 8.49 mm, maximum load: 6460 g

From the result that the values of *in vivo* strength test coincide well with the values of *in vitro* strength test, it can be said that the *in vitro* strength test reflects the erosion of the preparation in the stomach.

### (Comparative Example 2)

According to Example 1, flat circular tablets of gastric retentive preparation having a diameter of 8 mm were prepared, each containing: furosemide (50 mg), hydroxypropylmethyl cellulose 2208 (Metolose 90SH4000SR, 130 mg), and hydroxypropyl cellulose (20 mg) for the drug releasing layer; and ethyl cellulose (Ethocel STD10FP, 50 mg), dehydrogenated oil (Lubri Wax 101, 50 mg), and yellow ferric oxide (0.5 mg) for the gastric resident layer.

(Dissolution Test)
Small diameter (JP first fluid): 8.00 mm
Large diameter (JP second fluid): 8.00 mm
(Strength Test)
Small diameter (JP first fluid): 8.00 mm, maximum load: > 10000 9
(Confirmation Test for Gastric Residence Time)
Gastric residence time: 20 hours

### (Comparative Example 3)

According to Example 1, flat circular tablets of gastric retentive preparation having a diameter of 8 mm were prepared, each containing: furosemide (50 mg), hydroxypropylmethyl cellulose 2208 (Metolose 90SH4000SR, 130 mg), and hydroxypropyl cellulose (20 mg) for the drug releasing layer; and hydroxypropylmethyl cellulose 2208 (Metolose 90SH100000SR, 100 mg), and yellow ferric oxide (0.5 mg) for the gastric resident layer.

(Dissolution Test)
Small diameter (JP first fluid): 9.45 mm
Large diameter (JP second fluid): 7.02 mm
(Strength Test)
Small diameter (JP first fluid) : 7.15 mm, maximum load: 2570 g
(Confirmation Test for Gastric Residence Time)
Gastric residence time: 8 hours

### (Comparative Example 4)

According to Example 1, flat circular tablets of gastric retentive preparation having a diameter of 8 mm were prepared, each containing: furosemide (50 mg), hydroxypropylmethyl cellulose 2208 (Metolose 90SH4000SR, 130 mg), and hydroxypropyl cellulose (20 mg) for the drug releasing layer; and carboxyvinylpolymer (Carbopol 971P, 50 mg), polyethylene oxide (POLYOX Coagulant, 50 mg), and yellow ferric oxide (0.5 mg) for the gastric resident layer.

(Dissolution Test)
Small diameter (JP first fluid): 10.68 mm
Large diameter (JP second fluid): 19.28 mm
(Strength Test)
Small diameter (JP first fluid): 10.67 mm, maximum load: 2910 g
(Confirmation Test for Gastric Residence Time)
Gastric residence time: 10 hours

### (Comparative Example 5)

According to Example 1 except for using a flat circular die and punch having a diameter of 6 mm, flat circular tablets of gastric retentive preparation having a diameter of 6 mm were prepared, each containing: furosemide (50 mg), hydroxypropylmethyl cellulose 2208 (Metolose 90SH100000SR, 40 mg), and hydroxypropyl cellulose (10 mg) for the drug releasing layer; and ethyl cellulose (Ethocel STD10FP, 12.5 mg), hydrogenated oil (Lubri Wax 101, 12.5 mg), barium sulfate (25 mg), and yellow ferric oxide (0. 5 mg) for the gastric resident layer.

(Dissolution Test)
Small diameter (JP first fluid): 6.00 mm
Large diameter (JP second fluid): 6.00 mm
(Strength Test)
Small diameter (JP first fluid) : 6.00 mm, maximum load: 2020 g
(Confirmation Test for Gastric Residence Time)
Gastric residence time: 6 hours

As demonstrated above, a preparation according to the present invention has a sufficient gastric residence time and quickly disintegrates in the intestine, whereas the gastric residence time is not sufficient in the gastric retentive preparations of a swelling type of Comparative Examples 1, 3, and 4 due to small values of maximum load below 5000 g. The gastric residence time is not sufficient either in the gastric retentive preparation of a floating type of Comparative Example 5 due to its small diameter.

### INDUSTRIAL APPLICABILITY

A gastric retentive preparation according to the present invention has a sufficient gastric residence time, has a size that enables easy ingestion, and can disintegrate quickly after expelled from the stomach. The gastric retentive preparation is therefore useful as a sustained release preparation.

## Claims

1. A gastric retentive preparation comprising a gastric resident layer and a drug releasing layer, wherein the gastric resident layer does not disintegrate in the stomach and disintegrates in the intestine.

2. The preparation according to claim 1, wherein the gastric resident layer has a small diameter of 7 mm or more as measured after stirring the preparation in the first fluid at 200 rpm at 37°C for 15 hours under the conditions of the paddle method in the dissolution test in accordance with the Japanese Pharmacopoeia (hereinafter referred to as "JP"), and a large diameter of 6 mm or less as measured after subsequently stirring the preparation in the second fluid at 200 rpm at 37°C for 9 hours under the same conditions.

3. The preparation according to claim 2, wherein the preparation has a maximum load of 5000 g or more when compressed by 60 mm per minute to a thickness of 1 mm after being rotated at 200 rpm at 37°C for 10 hours in a horizontally laid 50 mL volume conical tube (30x115 mm) containing 50 g of glass beads having an outer diameter of about 4 mm to 5 mm and 30 mL of the JP first fluid, the JP first fluid (30 mL) being replaced every 2.5 hours during the rotation.

4. The preparation according to claims 2 or 3, wherein the gastric resident layer has a small diameter of 7 mm or more after being rotated at 200 rpm at 37°C for 10 hours in a horizontally laid 50 mL volume conical tube (30x115 mm) containing 50 g of glass beads having an outer diameter of about 4 mm to 5 mm and 30 mL of the JP first fluid, the JP first fluid (30 mL) being replaced every 2.5 hours during the rotation.

5. The preparation according to claim 1, wherein the gastric resident layer includes a polymer ingredient that has higher solubility or swellability in weakly acidic to weakly alkaline medium than in acidic medium.

6. The preparation according to claim 5, wherein the gastric resident layer includes 0 to 40 parts by weight of a hydrophobic ingredient selected from the group consisting of cellulose derivatives, acrylic acid copolymers, vinyl acetate polymers, oils and fats, higher fatty acids, higher fatty acid esters, higher alcohols, hydrocarbons, polycaprolactone, polylactate, polyglycolate, and polyamides, with respect to 1 part by weight of the polymer ingredient that has higher solubility or swellability in weakly acidic to weakly alkaline medium than in acidic medium.

7. The preparation according to claims 5 or 6, wherein the polymer ingredient that has higher solubility or swellability in weakly acidic to weakly alkaline medium than in acidic medium is one of an enteric ingredient and a polymer ingredient selected from the group consisting of carboxymethyl celluloses, polyacrylic acids, and polysaccharides having a carboxyl group or a sulfo group.

8. The preparation according to claim 7, wherein the enteric ingredient is selected from the group consisting of enteric cellulose derivatives, enteric acrylic acid copolymers, enteric polyvinyl derivatives, and enteric maleic acid-vinyl copolymers.

9. The preparation according to claim 7, wherein the gastric resident layer includes 1 to 40 parts by weight of a hydrophobic ingredient selected from the group consisting of cellulose derivatives, acrylic acid copolymers, vinyl acetate polymers, oils and fats, higher fatty acids, higher fatty acid esters, higher alcohols, hydrocarbons, polycaprolactone, polylactate, polyglycolate, and polyamides, with respect to 1 part by weight of the polymer ingredient selected from the group consisting of carboxymethyl celluloses, polyacrylic acids, and polysaccharides having a carboxyl group or a sulfo group.

10. The preparation according to any one of claims 5 to 9, wherein the preparation further includes a water-soluble ingredient selected from the group consisting of hydroxyalkyl celluloses, polyvinyl derivatives, polyethylene oxides, methyl cellulose, gelatin, polysaccharides, sugars, and sugar alcohols.

11. The preparation according to any one of claims 5 to 10, wherein the drug releasing layer includes hydroxyalkyl cellulose or polyethylene oxides.

12. The preparation according to any one of claims 5 to 11, wherein a drug contained in the drug releasing layer is one of: a drug that is absorbed in the duodenum and/or the jejunum; a drug that exhibits its effect in the stomach and/or the upper small intestine; a drug that is decomposed in the lower digestive tract, a drug that is susceptible to efflux into the digestive tract by drug transporters present in the intestinal cells in the lower digestive tract; and a drug that is insolubilized in an alkaline or neutral environment in the lower digestive tract.

13. A method for determining gastric resident properties of a gastric retentive preparation including a gastric resident layer and a drug releasing layer, wherein gastric resident properties are determined as desirable when the preparation has a large maximum load, and undesirable when the preparation has a small maximum load, as measured when the preparation is compressed by 60 mm per minute to a thickness of 1 mm after being rotated at 200 rpm at 37°C for 10 hours in a horizontally laid 50 mL volume conical tube (30x115 mm) containing 50 g of glass beads having an outer diameter of about 4 mm to 5 mm and 30 mL of the JP first fluid, the JP first fluid (30 mL) being replaced every 2.5 hours during the rotation.

14. The method according to claim 13, wherein gastric resident properties are determined as desirable when the preparation has a maximum load of 5000 g or more.

15. A method for controlling a gastric residence time of a gastric retentive preparation including a gastric resident layer and a drug releasing layer, wherein the method comprises controlling the maximum load set forth in the method of claim 13.
